# EUROPEAN PATENT APPLICATION

(11) **EP 4 763 119 A1**
(43) Date of publication of application: **24.06.2026**
(21) Application number: 24221830.3
(22) Date of filing: 19.12.2024
(51) Int. Cl.: A61B 34/20, A61B 34/30, A61B 34/32, A61B 90/11, A61B 90/00, A61B 90/50, A61B 34/00, A61B 17/00, A61B 34/10

(54) **AN IMAGE GUIDED SURGICAL ROBOT**

(71) Applicant: Luke Robotics, 38400 Saint Martin d'Heres (FR)
(72) Inventor: LENFANT, Jeremy, Saint Martin d Heres (FR); LAVALLEE, Stephane, Saint Martin d Heres (FR)
(74) Representative: Nordmeyer, Philipp Werner

(57) **Abstract**

The present disclosure generally relates to image-guided surgical robotic systems and methods of image-guided intervention that robotically actuate a medical tool along a trajectory for the purpose of reaching a target of interest.

## Description

### TECHNICAL FIELD

The present disclosure generally relates to image-guided surgical robotic systems, and methods of image-guided intervention, more particularly to image-guided surgical robotic systems and methods that robotically insert a medical tool along a trajectory reaching a target of interest.

### BACKGROUND

In recent years, robotics has significantly advanced medical imaging and interventional procedures, particularly in image-guided percutaneous needle placement. This technique is critical for diagnostic and therapeutic interventions such as biopsies, drug delivery, tumor ablation, and drainage. Tumor ablation can be performed using one or several needles delivering energy such as radio frequency, microwave, cryogeny, or the like. Percutaneous needle placement involves inserting a needle through the skin to reach a target in an organ or lesion, with imaging used to ensure precision. The introduction of surgical robotic systems into these procedures has transformed planning and execution, enhancing accuracy and reducing the risk of human error.

Surgical robotic systems have been developed to integrate with various imaging technologies, including ultrasound, computed tomography (CT), CT fluoroscopy (CTF), cone beam CT (CBCT), magnetic resonance imaging (MRI), and positron emission tomography (PET). Robots designed for percutaneous needle insertion assist primarily with planning the needle's trajectory and providing precise guidance to keep the needle aligned along the planned path. Despite these advances, needle insertion and advancement are still manual tasks performed by a clinician who controls the needle via a guide. While robotic systems improve guidance and planning, the need for manual control can introduce variability in precision based on the operator's skill and the complexity of the procedure.

Surgical robotic systems that not only guide the needle's trajectory but also insert and advance the needle into the target of interest robotically have been proposed. An example is detailed in Document WO202357587, which describes a patient-mounted, CT-guided, three-dimensional robotic system designed for percutaneous interventional procedures. The disclosed system begins by imaging patient to create a three-dimensional model of the target area, allowing the clinician to mark the target and select an optimal pathway for the needle. The clinician predetermines several checkpoints along the planned needle trajectory. These checkpoints, spaced apart by the clinician, are points where the system pauses to obtain a new scan and verify the needle's course, enabling course corrections if necessary. This process is repeated, with scans and corrections made at each checkpoint, until the needle successfully reaches the target. If the target is not reached, the trajectory is updated, and the needle insertion and scanning are repeated until the target is reached. This system incorporates a respiratory motion sensor, enabling needle advancement during a phase of patient respiration.

### DISCLOSURE OF THE INVENTION

During the acquisition of a medical image of a target of interest (e.g., tumor), the target of interest is typically in a particular phase of a biological cycle. These cycles include a variety of regular, rhythmic processes such as respiratory cycles (breathing), cardiac cycles (heartbeat), gastrointestinal motility cycles (digestive movements), or electromyographic (EMG) cycles in muscles (muscle activity patterns). Each of these cycles may lead to changes in the position and shape of the organs and tissues involved, thus the position of the target. After the image is acquired, and when a medical tool, such as a needle, is inserted into the body for diagnostic or therapeutic purposes, it is crucial that the organ is in the exact same phase of the biological cycle as it was during imaging. If the medical tool is inserted when the organ is in a different phase of a biological cycle, such as during a different point in the cardiac or respiratory cycle, the organ and the target of interest within it may have shifted. This discrepancy means that the medical tool may miss the target or reach it inaccurately, leading to procedural complications or less effective outcomes.

In addition to these regular, predictable cycles, non-periodic and unpredictable movements of the patient and/or medical equipment (e.g., robotic arm, end effector, needle) may further contribute to procedural inaccuracies. For example, vibration or a reflex action like a sneeze can cause abrupt and unanticipated displacement of tissues and devices, which may lead to misalignment of the needle trajectory.

Furthermore, during needle insertion, whether performed manually or robotically, the target organ can shift due to a range of factors such as tissue elasticity. Such movements can alter the alignment between the needle and the target, increasing the risk of misplacement.

One drawback of existing methods of image-guided interventions and surgical robotic systems is the slow procedural time. The time it takes for a needle to reach a target, such as a tumor, from an entry point, like the skin, typically ranges from several seconds to several minutes, with insertion speeds usually between approximately 10 mm/s and 40 mm/s. This significantly impacts on their ability to maintain accuracy in reaching the (moving) target of interest. When medical tool insertion is performed at this slow pace, the system allows more time for the target to undergo displacement due to the factors exemplified above. The longer the insertion process takes, the more opportunities there are for both regular biological rhythms and unpredictable movements of devices and/or patient to interfere with the precision of the tool's trajectory.

Another drawback of existing methods of image-guided interventions and surgical robotic systems is that the medical tool insertion is typically performed manually by a clinician, often requiring the patient to be under general anesthesia to minimize movement provided that the anesthesiologist voluntarily freezes patient's breathing between the image acquisition and the needle insertion or places the patient in the same instant of the respiratory cycle. However, general anesthesia causes obvious problems and may not consider other involuntary biological rhythms such as cardiac rhythms or digestive movements, or patient motion, which can still affect organ positioning. Additionally, techniques like instructing the patient to hold their breath at various stages of the procedure are often inadequate in stabilizing internal organs and may be inconvenient for both the patient and the physician. Since these biological cycles and movements of devices and/or patient are not fully accounted for, the accuracy of needle insertions is reduced, increasing the likelihood of missing the target.

Another drawback of the existing methods of image-guided interventions and surgical robotic systems is the potential need for reinsertion and navigation correction of the needle inside the patient if the initial attempt fails to reach the target due, for example, to organ movement, improper positioning of the needle, or movement of medical equipment. These procedures carry significant risks. For example, it can result in trauma to the surrounding tissues, causing unnecessary damage and prolonging the patient's recovery time. In addition, repeated attempts often involve additional imaging, which increases procedure time and radiation exposure for the patient and/or clinicians.

Another drawback of existing methods of image-guided interventions and surgical robotic systems that consider organ movement is that they are complex, unprecise, and involve a time-consuming registration process. For example, these methods and systems have proposed algorithms based on artificial intelligence (AI) and machine learning to consider breathing cycles. A key barrier to their implementation is the vast amount of data required to train AI algorithms effectively. These algorithms need to be trained on extensive medical image datasets that represent diverse anatomical structures, imaging devices, and biological conditions specific to each patient. Obtaining such datasets is challenging, as it requires radiologists and other specialists to carefully and continuously label and curate the images. In addition, these systems are complex and are time consuming to register images, calculate trajectories and insert a medical tool and they may not be reliable.

Another drawback of existing patient-mounted robots designed for automatically inserting a needle is their limited functionality, especially when multiple needle insertions are required. These systems are often restricted by their small workspace. This means that the robotic system can only operate around the initial insertion point, making it difficult to perform procedures that require multiple insertions at varying angles or locations. Moreover, patient-mounted robots need to be manually prepositioned near the target's entry point before the procedure begins. If there is a misjudgment in identifying the target location, the robot must be repositioned, which often necessitates additional CT scans to re-evaluate the new target, resulting in increased radiation exposure and procedural time. Another problem is the robot's attachment to the patient. Stable fixation of the robot on the patient's body, particularly on the skin, is required for maintaining precision. Any instability or inadequate attachment can cause the robotic system to shift during the procedure, leading to a loss of accuracy in needle placement. This instability not only compromises the success of the procedure but can also result in complications if the target is missed.

Another drawback of using intraprocedural correction (i.e., correction of needle path after the needle is inserted into a patient) and selecting checkpoints along the needle trajectory is the increase in procedural time and exposure risks. While these checkpoints can help correct deviations, each one requires pausing the procedure to assess the needle's alignment. This necessitates additional imaging scans to verify the needle's position. These repeated scans not only prolong the procedure but also expose the patient, physician, and medical staff to higher levels of radiation, increasing the risk of radiation-related complications. Furthermore, intraprocedural corrections may only be feasible for certain tissues due to the anatomical and physiological constraints of specific organs. For instance, in lung procedures, intraprocedural correction may cause puncturing of the pleura (the membrane surrounding the lungs) and increasing the risk of pneumothorax (collapsed lung).

Therefore, it would be desirable to provide alternative methods of image-guided intervention and surgical robotic systems to address at least one or more of the above-mentioned drawbacks.

To solve one or more of the problems mentioned above, the present disclosure provides methods of image-guided intervention and surgical robotic systems. Specifically, the present disclosure discloses methods of image-guided intervention and surgical robotic systems designed to mitigate the effects of movements, such as biological rhythms, as well as movements of a subject, surrounding tissues, target, and/or medical equipment used in the procedure (e.g., end effector, medical tool, robotic arm), on the accuracy of targeting.

Additionally, the methods of image-guided intervention and surgical robotic systems disclosed herein enable reaching a (moving) target without the need for continuous monitoring, optimizing, or steering of a medical tool inside the patient. For example, according to the methods and surgical robotic systems of present disclosure, a needle is inserted once along a trajectory, and it reaches the target of interest accurately without a need for verification of the needle's path at various checkpoints along the trajectory. This approach results in reduced radiation exposure for both the patient and clinicians and reduces the procedural time reaching the target as described above.

Methods of image-guided intervention and surgical robotic systems according to any one of the embodiments of present disclosure provide series of pre-medical tool insertion processes, which rapidly and reliably aligns the medical tool along a trajectory reaching the (moving) target.

Additional features and advantages of the invention will become apparent from the following description and claims.

The embodiments summarized below in this section are intended to illustrate certain aspects of the invention and are not to be construed as limiting. Additional embodiments, features, and variations will be apparent to those skilled in the art from the figures, detailed description, and claims that follows. It will be understood by those skilled in the art that the invention encompasses additional embodiments, modifications, and variations that may not be explicitly described in this section. Rather, further embodiments, features, and methods are described throughout this application and are within the scope of the invention. All such embodiments are intended to fall within the scope of the invention, and any combination of features disclosed in different embodiments should be considered.

According to the first aspect of the present disclosure, there is provided a method, for example, a method of image-guided interventional radiology. Another example, a method for robotically actuating a medical tool along a trajectory extending from an entry point to a target of interest within a subject is provided. The following exemplary embodiments of the method of present disclosure are disclosed:

In an embodiment, the method comprises: receiving a first medical image data from an imaging device, wherein the first medical image data represents a first volume of the imaged subject, comprising a target within a subject. Optionally, the first medical image data further comprises a reference marker. The method further comprises maintaining alignment of a medical tool along a trajectory extending from an entry point on subject's surface to the target, wherein the medical tool is positioned at the entry point while aligned with the trajectory; receiving a second medical image data from the imaging device while the medical tool maintained its position at the entry point aligned with the trajectory, wherein the second medical image data represents a second volume of the imaged subject, comprising the target and a reference marker; instructing the end effector to insert the medical tool along a new trajectory extending from the entry point on the subject's surface to the target upon receiving an insertion instruction, wherein the new trajectory is determined based on the second medical image.

In an embodiment, the method further comprises positioning and orienting, by one or more processors, the medical tool at the entry point.

In an embodiment, the second volume of the imaged subject is smaller than the first volume of the imaged subject.

In an embodiment, the method comprises maintaining alignment of the medical tool along the trajectory and/or the new trajectory by an applied force exerted by the end effector on the subject's surface while the medical tool is positioned at the entry point, optionally, above the entry point.

In an embodiment, the method further comprises instructing, via one or more processors, the end effector to pivot the medical tool at the entry point to align with the new trajectory reaching the target in the second medical image data.

In an embodiment, the method further comprises instructing, via one or more processors, the end effector to maintain an applied force to subject's surface while pivoting the medical tool at the entry point.

In an embodiment, the method further comprises receiving, by one or more processors, identification data for the target and the entry point based on the first medical image data and determining, via one or more processors, the trajectory between the identified target and the entry point.

In an embodiment, the method further comprises receiving, by one or more processors, identification data for the target; determining, by the one or more processors, a trajectory extending from the target to an external point on the subject, wherein the external point is identified as an entry point.

In an embodiment, the method further comprises registering, via one or more processors, coordinate system of the second medical image data with coordinate system of the first medical image data and determining a new trajectory from the target in the second medical image data and the entry point.

In an embodiment, the method further comprises registering the coordinate system of the reference marker in the first medical image data and the second medical image data with the coordinate system of the end effector.

In an embodiment, the reference marker comprises an emitter or receiver trackable by a tracking system and an object, for example, radiopaque materials, that is visible in both the first and second medical images. The object can have any visible shape and size such as tubes, rods, or spheres. Optionally, the tracking system is an electromagnetic tracking system or optical tracking system. Optionally, the emitter and/or receiver is part of a sensor.

In an embodiment, the method further comprises instructing a tracking system to track the reference marker and the end effector with respect to each other. Optionally, the reference marker visible in the first and/or the second medical image has an emitter and/or receiver, and it is linked to the patient. Optionally, the reference marker having the emitter and/or receiver is linked to the end effector. Optionally, the reference marker is rigidly or non-rigidly linked to the patient and/or the end effector.

In an embodiment, the medical tool is a needle. Optionally, the medical tool is a canula for inserting, for example, an ablation needle. Optionally, the medical tool is an ablation needle. Optionally, the needle is a biopsy needle.

In an embodiment, the reference marker visible in the first medical image data and the second medical image data is attached to the end effector or is part of material of the end effector.

In an embodiment, the reference marker in the first medical image data and the second medical image data is registered to the end effector via an intermediate referential.

In an embodiment, the first and the second medical image data are three-dimensional medical image data.

In an embodiment, two or more medical tools are actuated, optionally, inserted and one or more processors determine the order in which the medical tools are to be actuated or inserted.

In an embodiment, the method further comprises calculating and determining, by one or more processors, a path of the medical tool to be aligned with the new trajectory.

In an embodiment, the method further comprises receiving instructions from a user interface communicatively coupled with a display. The display is configured to display at least one or more of the following: the first medical image data, the second medical image data, the trajectory, the new trajectory, target and entry point.

According to the second aspect of the present disclosure, there is provided a non-transitory computer readable medium. The non-transitory computer readable medium storing computer program instructions which, when executed by one or more processors, cause the one or more processors to perform a method according to any one of the preceding embodiments and any one of the embodiments presented herein.

According to the third aspect of the present disclosure, there is provided a surgical robotic system. The surgical robotic system of present disclosure is configured to robotically actuate (e.g. insert) a medical tool along a trajectory reaching a target of interest during, for example, image-guided interventional procedure. The following exemplary embodiments of the surgical robotic system of present disclosure are disclosed:

In an embodiment, the surgical robotic system comprises an end effector configured to hold and actuate (e.g. insert) at least one medical tool; and a computing device communicatively coupled to the end effector, wherein the computing device comprises a non-transitory computer readable medium storing computer program instructions which, when executed by one or more processors, cause the one or more processors to perform a method of any one of the preceding embodiments and any one of the embodiments disclosed herein.

In an embodiment, the end effector has three or less degrees of freedom. Optionally, the end effector has a pivoting mechanism to pivot a medical tool. Optionally, the end effector has a force sensor to control an applied force by the medical tool to the entry point.

In an embodiment, the surgical robotic system comprises a robotic arm connected to the end effector. Optionally, the robotic arm has six degrees of freedom, it can however have more or less than 6 DoF.

Methods and surgical robotic systems according to any of the embodiments of the present disclosure allow for a rapid procedure time from the moment the second medical image is received, the target is identified, a new trajectory is generated, and the end effector is instructed to insert a medical tool robotically along the new trajectory.

The above-described aspects, features, advantages, and further aspects of the present disclosure will become more apparent from the following disclosure and drawings.

### BRIEF DESCRIPTION OF DRAWINGS

The features and advantages of the present disclosure will be more apparent considering the embodiments below, which are provided by way of illustration and in no way are limiting:
FIG. 1 shows a perspective view of an exemplary surgical robotic system.
FIG. 2 shows another perspective view of an exemplary surgical robotic system with an end effector inside the gantry of an imaging device. The imaging device obtains the first medial image (CT1) and the end effector having a reference marker is within the volume of the first medical image.
FIG. 3 shows another perspective view of an exemplary surgical robotic system with an end effector outside of the gantry after the first medical image is obtained.
FIG. 4 shows another perspective view of an exemplary surgical robotic system where a trajectory from an entry point to a target is determined after obtaining the first medical image.
FIG. 5 shows another perspective view of an exemplary surgical robotic system where the medical tool is positioned and oriented along the trajectory from an entry point to the target after the first medical image is obtained.
FIG. 6 shows another perspective view of an exemplary surgical robotic system where the imaging bed together with the end effector moves inside the gantry, while maintaining the position of medical tool at the entry point along the trajectory reaching the target. As illustrated, the target and the reference marker are within the volume of CT2.
FIG. 7 shows another perspective view of an exemplary surgical robotic system where the medical tool pivots at the entry point after obtaining the second medical image (CT2) to align with new trajectory.
FIG. 8 shows another perspective view of an exemplary surgical robotic system using a tracking system. As illustrated, the first medical image (CT1) is obtained. CT1 includes target and a reference marker comprising an emitter or receiver detectable by a tracking and an object detectable on the CT1.
FIG. 9 shows another perspective view of an exemplary surgical robotic system using a tracking system. As illustrated, after obtaining CT1, the patient slides outside of the gantry.
FIG. 10 shows another perspective view of an exemplary surgical robotic system using a tracking system. As illustrated, a trajectory from an entry point to a target is determined after obtaining CT1.
FIG. 11 shows another perspective view of an exemplary surgical robotic system utilizing a tracking system. As illustrated, the medical tool is positioned and oriented along the trajectory from an entry point to the target after CT1 is obtained.
FIG. 12 shows another perspective view of an exemplary surgical robotic system using a tracking system. As illustrated, the imaging bed together with the end effector moves inside the gantry, while maintaining the position and orientation of medical tool at the entry point along the trajectory reaching the target. As illustrated, the target, the entry point, and the reference marker are inside CT2.
FIG. 13 shows another perspective view of an exemplary surgical robotic system using a tracking system. As illustrated, the imaging bed moves inside the gantry. The end effector remains outside of the gantry while maintaining the position and orientation of medical tool at the entry point along the trajectory reaching the target. As illustrated, the target and the reference marker are inside CT2. The entry point is outside CT2.
FIG. 14 shows another perspective view of an exemplary surgical robotic system using a tracking system. As illustrated, the imaging bed moves inside the gantry. The end effector remains outside of the gantry while maintaining the position and orientation of medical tool at the entry point along the trajectory reaching the target. As illustrated, the target, the intermediate referential, and the reference marker are inside CT2. The entry point is outside CT2.
FIG. 15 is an exemplary flowchart illustrating a process of image-guided intervention.
FIG. 16 is another exemplary flowchart illustrating a process of image-guided intervention.
FIG. 17 shows a perspective view of an exemplary end effector having a semi-spherical support arm.
FIG. 18 shows an exemplary schematic diagram illustrating a computing device suitable for implementing the surgical robotic system and methods of present disclosure.

### DETAILED DESCRIPTION OF EMBODIMENTS

The embodiments disclosed herein are illustrative of the principles of the invention and are not intended to limit the scope thereof. Those skilled in the art will appreciate that the features, configurations, and functions described with respect to particular embodiments may be applied to other embodiments and in various combinations without departing from the invention's scope. Unless expressly stated otherwise, each feature disclosed in connection with one embodiment is applicable to other embodiments as well, in any suitable combination, to achieve desired results. Modifications, alterations, and adaptations to the embodiments described herein may be made, as will be apparent to those skilled in the art, without departing from the spirit or scope of the present disclosure.

In the following description, reference is made to the accompanying drawings which illustrate several embodiments of the present invention. It is understood that other embodiments and features disclosed herein may be utilized.

The term "intervention" as used herein refers to any manipulation of a target of interest, for example, for diagnostic and/or therapeutic purposes. The diagnostic and/or therapeutic purposes include biopsies, fluid aspiration and drainage, catheterization, tumor removal, tumor ablation, stent placement and angioplasty, endovascular embolization, percutaneous drainage of abscesses, fracture repairs, orthopedic fixation, pain management (e.g., radiofrequency or cryoablation), and drug delivery.

The term "image-guided intervention" as used herein refers to a procedure in which an imaging device is employed, either directly or indirectly, to assist or guide the intervention.

As used herein, the term "imaging device" generally refers to a device which can generate a one-dimensional or a multidimensional such as a two-dimensional, a three-dimensional, a four-dimensional, a five-dimensional, a six-dimensional image of a subject or of a part thereof. The imaging device may be a CT, CTF, CBCT, MRI, C-Arm Fluoroscopy, Positron Emission Tomography (PET), Intraoperative CT (iCT), Intraoperative MRI (iMRI), 3D endoscopic camera, or Optical Coherence Tomography (OCT). The imaging device can integrate with other systems to support the functions of the methods and surgical robotic systems of present disclosure. For instance, the imaging device can integrate with tracking systems (not shown), such as optical tracking, or electromagnetic tracking systems.

The term "subject" generally may be chosen from a living subject and a non-living subject. For example, the subject can be a human or an animal. The subject may comprise one or more parts of a living or non-living subject, such as one or more body parts of a human or an animal.

The term "target" as used herein includes, but is not limited to, a feature of a subject like an anatomical feature of a subject that a medical tool intends to interact with, for example, for diagnostic and therapeutic purposes. The target can, for example, be a blood vessel and cardiovascular structures (e.g., coronary arteries, peripheral arteries, cardiac valves, arteriovenous malformations), tumors and cancerous lesions (e.g., liver tumor, kidney tumor, lung nodules and tumors, brain tumor, breast tumor, spinal tumor), joints and spine (e.g., spinal never, joints, vertebrae, herniated discs), gastrointestinal and hepatobiliary system (e.g., liver, spleen, pancreas, colon, gallbladder), central nervous system (e.g., spinal cord, brain), kidney and urinary system (e.g., kidney stones, ureter, bladder), lung and respiratory system (e.g., lung nodules or masses, pleural space, lung tumors, trachea and bronchi), musculoskeletal system (e.g., bones and soft tissues), lymphatic system (e.g., lymph nodes), reproductive system (e.g. ovarian cysts or tumors, uterus and ovaries).

The term "reference marker" as used herein refers to any object that is identifiable or visible in the medical image and/or any object such as emitter, receiver, and sensor detectable by a tracking system. For example, objects visible in the medical image include radiopaque materials. Examples of radiopaque material include platinum, titanium, tantalum, stainless steel, gold, barium sulfate, radiopaque ceramics, and iodine-based materials. In another example, sensor includes receivers and/or emitters detectable by tracking systems such as optical, electromagnetic (EM), RFID based technology and ultrasonic tracking systems. A sensor can also refer to a device or component that detects or emits a signal (such as infrared light, electromagnetic waves, or ultrasound) to track its position and orientation. For example, the sensor can be an active sensor (LEDs that emit infrared light) or passive sensors (reflective spheres). For example, the sensor includes elements that emit or detect ultrasound signals. In EM Tracking Systems sensors detect electromagnetic fields and provide positional data, while emitters generate the EM field for tracking purposes.

In another example, the reference markers disclosed herein can comprise an emitter or receiver detectable by a tracking system and an obj ect visible in medical image by an imaging device such as radiopaque material. An example of such reference marker is described in U.S. Pat. No. 8,611,985, the disclosure of which is herein incorporated by reference. In this example, the reference marker comprises a magnetic emitter or receiver having at least two coils arranged in a manner to define at least two vectors in a coordinate system of the magnetic tracking system and a geometric element comprising hollow plastic tubes visible on image data of the imaging device and having a unique geometry detectable by the imaging device.

The reference markers disclosed herein can be in any shape and size such as spherical balls, tubes, or rods.

The term "medical tool" as used herein broadly encompasses a tool, an instrument, a device or the like used to reach the target of interest. For example, any tool, instrument, or device used for diagnostic and/or therapeutic purposes. The medical tools according to the present disclosure include needles, cannulas, drills, guidewires, stents, balloon catheters, ablation probes (e.g., radiofrequency or microwave), trocars, biopsy forceps, biopsy needles, embolization coils, snare devices, vascular filters, endoscopic instruments, coagulation probes (laser, plasma, and the like), aspiration devices, and other devices suitable for performing the surgery or intervention.

As used herein, the term "surgical robotic system" refers to a system configured to assist in an image-guided interventional procedure. For example, the surgical robotic system of present disclosure is configured for use in an image guided surgical intervention performed on a subject for diagnostic or therapeutic purposes. The surgical robotic system of present disclosure can also be used in other surgical and non-surgical applications, as will be appreciated by a person skilled in the art.

As used herein, the term "entry point" refers to a point on the surface of a subject (such as a patient's skin) where a medical tool is initially contacted to gain access to internal anatomical structures of the subject and a target. The entry point serves as the initial point of contact between the medical tool and the subject. The entry point can be a predefined point or a dynamically determined point on the surface of a subject. The term "dynamically determined" includes, but is not limited to, a process that is adjusted or calculated based on changing conditions or information gathered during the procedure. For example, when the condition or information indicates changes in the position of the target or surrounding anatomy, the system may adjust the entry point to maintain precision and safety. For example, one or more processors of the surgical robotic system of the present disclosure receive information about the location of the entry point from, for example, a display. For example, a clinician identifies the position of the entry point on the subject from a medical image displayed on a monitor and the processors receives the positional data of the entry point. In another example, one or more processors determine the entry point, which is aligned with the calculated trajectory to the target.

The phrase "Medical tool is positioned at the entry point" or similar phrase refers to a medical tool being in contact (i.e. directly touching) with the surface of the subject at a specified location identified as the entry point. This contact with the entry point includes alignment of the medical tool along a trajectory toward a target but excludes any penetration of the subject's surface unless expressly stated otherwise.

As used herein, the term "robotically" includes, but not limited to, being capable of performing a function (e.g., inserting a medical tool from an entry point along a trajectory reaching a target of interest) autonomously, without human intervention. For example, the surgical robotic system of present disclosure performs the insertion of a needle to a target autonomously by receiving instructions from a computing device having one or more processors.

As used herein, the term "actuate" or "actuation" includes, but not limited to, inserting, moving, exerting a force on, and other similar actions alone or combinations thereof. For example, one or more processors instruct the end effector to actuate a medical tool includes, but is not limited to, robotically insert a medical tool such a needle along a trajectory that reaches a target. In this example, the insertion is performed without manual insertion by a clinician like a surgeon or a radiologist. Additionally, if desired, the actuation mechanism can also be configured to subsequently return the medical tool from the insertion position to its initial position. The actuation mechanism can also be configured to release the medical tool after insertion or after return instruction.

As used herein, the term "trajectory" refers to a path desired and/or intended to be followed. A trajectory may be modeled and graphics representing the trajectory may be displayed on a display screen. A trajectory may be a linear trajectory (e.g., wherein all points along the trajectory fall onto a line) or a non-linear trajectory.

As used herein, the term "position" refers to the location of an object (e.g., medical tool, target, entry point) or a portion of an object in a three-dimensional space (e.g., three degrees of translational freedom along Cartesian X, Y, Z coordinates). The term "positioning" includes placing an object (e.g., medical tool, end effector) or a portion of an object in a three-dimensional space (e.g., three degrees of translational freedom along Cartesian X, Y, Z coordinates).

As used herein, the term "orientation" or "orient" and the like refers to the rotational placement of an object (e.g., medical tool, end effector) or a portion of an object in a three-dimensional space (e.g., three degrees of rotational freedom-e.g., roll, pitch, and yaw).

As used herein, the terms "pivot," "pivoting," and similar terminology refer to the movement of an object (e.g., a medical tool) around a fixed point of contact. For example, when a medical tool, such as a needle, pivots at an entry point (e.g., a point on the patient's skin), the point of contact on the patient's skin serves as the pivot. During this movement, the angle and/or orientation of the medical tool changes relative to this fixed point, while the point of contact itself remains stationary.

As used herein, the terms "tracking system" and "localization unit" are used interchangeably and broadly encompass all tracking systems known for tracking objects in coordinate space. For example, the tracking system is used to track the position and/or orientation of a medical tool, end effector, and/or robotic arm during the surgical or interventional procedure. The tracking systems include Electromagnetic (EM) tracking systems, Optical tracking systems, Ultrasound tracking systems, Inertia Measurement Units (IMUs), Camera-Based or Vision-Based systems, Radiofrequency (RF) tracking systems, or hybrid tracking systems where two or more tracking systems are used.

The term "registration" or "registering" or the like refers to the spatial transformation relating to two or more coordinate systems (e.g. end effector coordinate system, tracking system coordinate system, image coordinate system). The registration may be a pose between two coordinate systems, and may be expressed, and represented in computer memory, according to any convenient convention (e.g. vectors, rotation matrices, transformation matrix, Euler angles, quaternions, etc.). Registration may be performed via an intermediate referential.

The term "intermediate referential" as used herein includes, but is not limited to, a coordinate system or a reference frame that acts as a bridge between two or more coordinate systems. For example, the intermedial referential enables alignment and translation of positional data from one coordinate system to another, ensuring that two or more devices (for example, devices from different systems) are accurately tracked with respect to each other.

The term "communicatively coupled" includes, but is not limited to, communicating control signals by which one element may direct or control another. For example, one or more processors direct or control the movements and actions of the imaging device, robotic arm, and end-effector. Further, the term includes, but is not limited to, that two or more elements may be in contact with one another by any means of communication including through a wire or other interconnect connection, through a wireless communication or link, and/or the like.

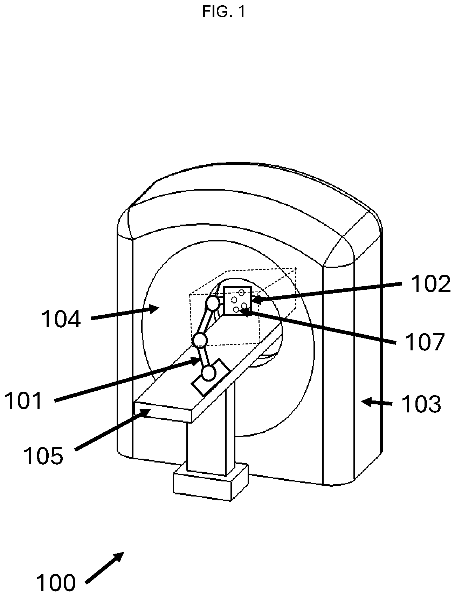

FIG. **1** shows a schematic diagram illustrating the surgical robotic system **100** according to an embodiment of the present disclosure.

In this exemplary embodiment, the surgical robotic system of present disclosure includes one or more processors (not shown), a robotic arm **101,** an end effector **102** configured to hold a medical tool. In the description that follows, surgical robotic system **100** is configured to perform the methods of present disclosure.

The one or more processors of the surgical robotic system of present disclosure are communicatively coupled to an imaging device **103,** a robotic arm **101,** and an end-effector **102.**

As shown in FIG. **1****,** the imaging device **103,** for example, includes a gantry **104** that contains an x-ray tube, and a bed **105,** which can be moved into the gantry **104** while the x-ray tube rotates around a subject (e.g., a patient) on the bed **105.**

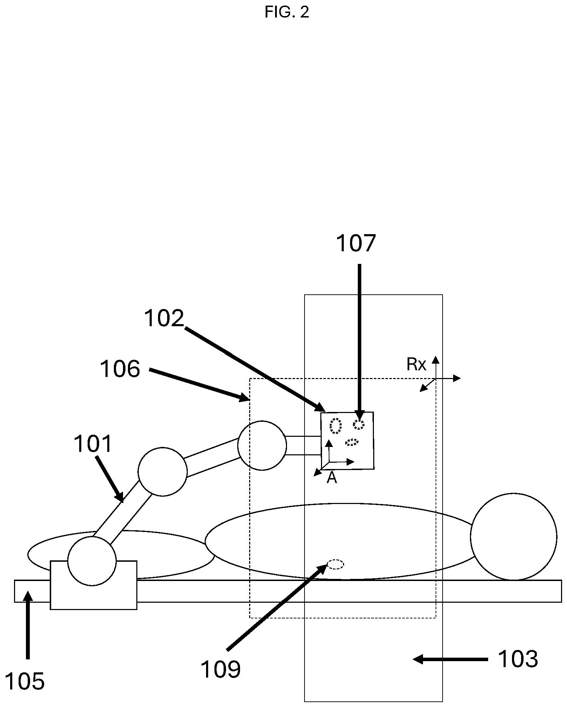

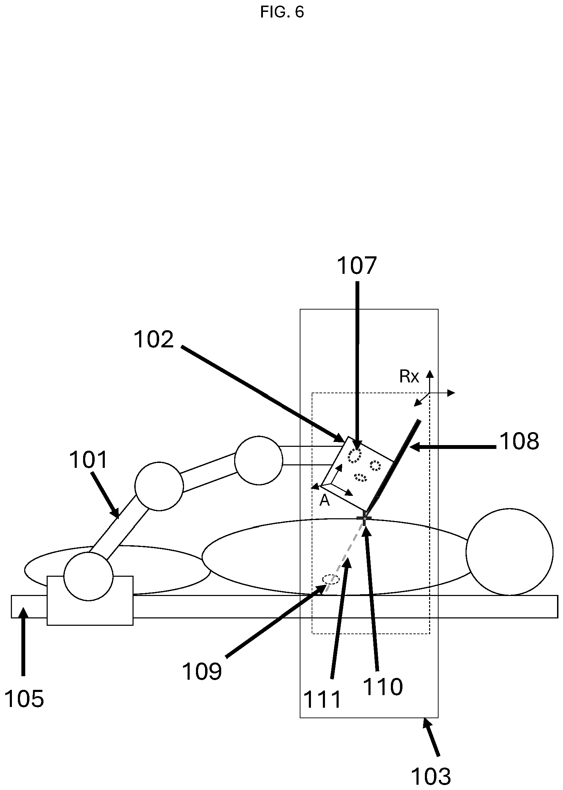

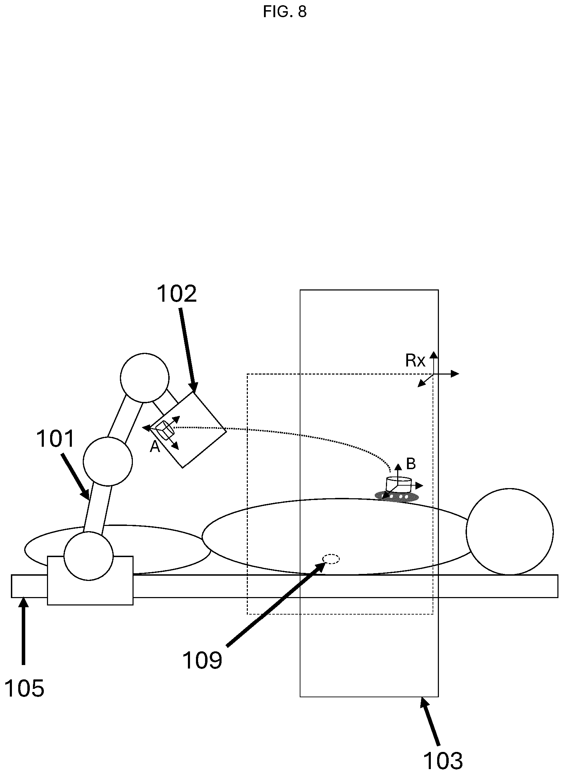

In an embodiment, the surgical robotic system of present disclosure is mounted on the imaging device's bed and moves with the bed inside and outside of the gantry. For example, as shown in FIG. **2** and FIG **6****,** all or part of the end effector can enter the gantry when the imaging device obtains the medical images. Optionally, as shown in FIG. **8** and FIG. **12****-14,** the end effector is positioned outside the gantry when the imaging device obtains the medical images. The position and location of the robotic arm and the end effector can be changed based on, for example, instructions received from one or more processors of the robotic surgical system, and it depends, for example, on the required angle between the medical tool (such as a needle) and the entry point to reach the target of interest.

In an embodiment, the robotic arm of the surgical robotic system of present disclosure may have one or more joints providing a plurality of degrees of freedom (DoF) in operating and controlling the movement of the end effector. For example, robotic arm may have up to six DoF. For example, the robotic arm may have at least 2, 3, 4, 5, 6, 7 or 8 DoF. For example, the robotic arm has a 6 DoF. Having 6 DoF allows the robotic arm to position and orient itself with freedom in the workspace and around the patient, mimicking the range of motion a human arm in three-dimensional space might have. 6 DoF includes three translational (linear) movements and three rotational (angular) movements. The translational movement are movements along the three Cartesian axes including X-axis (Forward/Backward), Y-axis (Left/Right), and Z-axis (Up/Down). The Rotational movements describe rotations around each of the three Cartesian axes such as Roll (Rotation about X-axis), Pitch (Rotation about Y-axis), and Yaw (Rotation about Z-axis).

Optionally, the robotic arm operates based on commands from the computing device (or control unit) having one or more processors.

In an embodiment, the surgical robotic system of present disclosure includes an end effector. The end effector of the present disclosure is designed to hold a medical tool and to robotically actuate (e.g. insert) a medical tool along a trajectory from an entry point to a target. Optionally, the end effector's functions such as orienting, positioning, pivoting, and actuating (e.g., inserting) of a medical tool is controlled by one or more processors.

In an embodiment, the end effector of present disclosure comprises a medical tool holding mechanism to hold and to release a medical tool. For example, the end effector includes a clamp or collet mechanism to firmly hold the medical tool and allow its release when instructed by one or more processors or manually by an operator.

In an embodiment, the end effector of present disclosure comprises a pressure application pad or a spring-loaded mechanism near the tip of a medical tool in contact with the entry point (e.g. near the needle tip), enabling the device to press against the surface of the entry point (e.g. skin) without puncturing it. The pad distributes the force evenly across the contact area, preventing localized pressure spikes that could unintentionally penetrate the surface (e.g. skin).

In an embodiment, to monitor and control the force exerted by the end effector on the subject, the method and the robotic system of present disclosure include force-monitoring based on the electrical current consumed by a robotic system's motor. The current drawn by the motor is directly correlated with the torque output of the motor, which in turn is linked to the force applied by the end effector on the subject's surface.

In an embodiment, the end effector of present disclosure comprises force/torque sensors to continuously monitor the force applied to the surface of the entry point (e.g. skin). Feedback from these sensors allows the system to adjust if the applied force nears a threshold that could lead to puncturing. For example, placing force/torque sensors near the needle's tip or within the end effector allows monitoring of the force exerted on the skin. Feedback from the force/torque sensor is processed by control algorithms (such as PID controllers) that adjust the applied force based on the sensor's readings, ensuring it remains below the puncture threshold.

In an embodiment, the end effector of present disclosure can comprise adaptive control algorithms such as adaptive impedance control to adjust the end effector's force based on the type of the entry point (e.g. type of tissue or skin the needle is interacting with). For example, the system detects resistance from the skin and dynamically modules the force.

In an embodiment, the robotic surgical system of present disclosure comprises high-resolution cameras with image processing algorithms that can track the indentation or deformation of the entry point (e.g. a point on the skin of a patient). By estimating deformation, the system can indirectly measure the force being applied and adjust the medical tools or needle's pressure to stay within limits.

In an embodiment, the end effector of the present disclosure is configured to pivot the medical tool such as a needle at an entry point (e.g. a point on the skin of a patient). This allows for angular adjustments around a fixed contact point, enabling the tool to reach various trajectories reaching the target without repositioning the entry point. For example, the end effector comprises a gimbal-like joint or a spherical bearing that allows multi-directional pivoting. A gimbal mount or similar structure would let the end effector hold the medical tool such as the needle at an adjustable angle while maintaining contact at the same entry point, allowing for manipulation in various directions.

In an embodiment, the end effector of present disclosure provides a pivot point on the entry (e.g., on the skin of a patient) with controlled force to stabilize the medical tool at the point of entry and to avoid puncturing surface of the entry point (e.g. puncturing the skin) while pivoting. For example, the end effector comprises force sensors that adjust pressure as the angle of the medical tool changes, ensuring a balanced distribution of force around the pivot point. For example, multi-axis force/torque sensors can measure the applied forces and moments (torque) around the pivot point. These sensors enable control over both the force applied to the entry point (e.g., a point on the skin) and the pivoting force, allowing the end effector to adjust its angle without exceeding the pressure threshold that would puncture the entry point.

In an embodiment, the medical tool such as a needle is positioned and oriented at the entry point on the patient's skin. Force sensors communicatively coupled with the end effector at the pivot point ensure that a consistent pressure is maintained on the skin without puncture. As a processor receives input about a new trajectory reaching the target from an entry point, the end effector adjusts the angle of the needle by activating actuators in the gimbal or pivot mechanism. Throughout this process, force feedback is continuously monitored adjusting any excess force on the skin. The control algorithms, via one or more processors, ensure that the pivot angle aligns with the new trajectory, adjusting based on position and force data. Thus, end effector can pivot accurately around a fixed skin contact point, applying controlled pressure while adjusting the needle's angle to align with the new trajectories.

In an embodiment, the end effector of present disclosure may have a plurality of DoF in operating and controlling the movement of one or more medical tools. For example, the end effector may have up to six DoF. For example, the end effector may have at least 2, 3, 4, 5, or 6 DoF. For example, the end effector has a 3 DoF. The end effector with 3 DoF reduces the complexity of the system and speeds up the medical tool actuation. For example, in certain medical procedures, like needle insertion for biopsies, injections, or minimally invasive surgeries, the task may require high precision in one or two linear movements (e.g., inserting along a straight line). The end effector with 3 DoF results in a faster and more direct insertion. Each additional degree of freedom may require extra control and planning. An end effector with a 3 DoF can have less processing load on the processor.

In addition, by limiting the range of movements (e.g., 2 or 3 DoF), the surgical robotic system of present disclosure reduces the risk of unintended or unsafe actions that might occur during the procedure. This can stabilize the needle or other medical tools when they are actuated or when they are inside the subject. For example, 2 or 3 DoF ensures that the medical tool such as a needle does not inadvertently shift or translate in any direction, which could potentially cause trauma to the surrounding tissue or lead to inaccurate placement.

In an embodiment, the end effector that pivots a medical tool at a fixed entry point has 2 or 3 DoF. For example, an end effector configured to pivot around a single fixed point has 2 DoF. This means the tool can pivot around two perpendicular axes that intersect at the fixed point, allowing full orientation control relative to that point. In another example, the end effector that pivots the medical tool at a fixed entry point has 3 DoF and is configured to additionally allow for axial rotation (twisting around the medical tool's own axis).

In one embodiment, the surgical robotic system of present disclosure includes a robotic arm having multiple DoF such as 6 DoF and an end effector having a 3 or less DoF. This combination allows flexible movement within the workspace and around the patient in addition to precise pivoting, angling and/or positioning at the entry point. Furthermore, this designed combination allows for safer and faster processing time by a processor to send an instruction to the end effector or its actuator mechanism to insert a medical tool such as a needle along the trajectory.

In an embodiment, the end effector is connected to one end of the robotic arm. Optionally, the robotic arm and the end effector are integrated as one unit, therefore the terms "robotic arm" and "end effector" are used interchangeably in this embodiment. For example, the robotic arm can perform all the functions of the end effector disclosed herein. For example, some parts of the robotic arm can have 6 DoF and the part of the robotic arm holding a medical tool has 3 or less DoF. Robotic arm and the end effector can be separated or connected by, for example, hinges or any other device that provide the capability to the robotic arm and the end effector in achieving the mentioned DoF and function.

In an embodiment, the end effector comprises a support arm positioned adjacent to the entry point on the surface of the subject. The support arm provides stability, controls pressure and enables precise alignment of the medical tool at the entry point with the target. Optionally, the support arm comprises a pivot mechanism, allowing for rotational adjustments to align the medical tool precisely with the trajectory extending from the entry point to the target. The support arm can be configured in any shape and size to provide the best contact with the surface of the subject.

In one embodiment, the end effector comprises a support arm positioned adjacent to the entry point on the subject's surface such as skin. Optionally, this arm includes a pressure sensor, continuously monitoring the applied force to prevent premature puncture. The pressure sensor provides feedback to a control processor, which dynamically adjusts the applied force to ensure that the medical tool remains stable at the entry point without penetrating the surface of the subject (e.g., skin) until insertion is instructed.

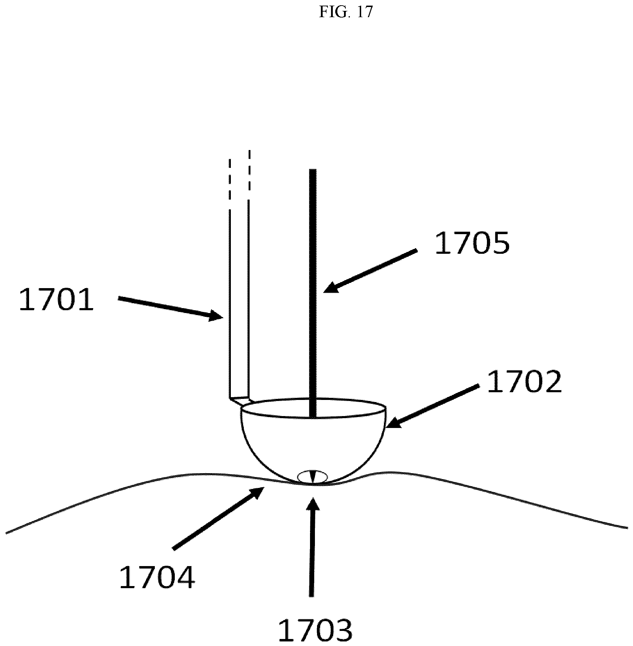

As shown in FIG. **17**, in one embodiment, the end effector comprises a support arm **1701** having a semi-spherical base **1702** surrounding the entry point **1703** and is contact with surface of the subject **1704,** here skin of a patient. Medical tool **1705,** such as a needle or a biopsy needle through a hole at the center of the base is in contact with the entry point on the surface of the subject. The semi-spherical support structure that surrounds the entry point creates a uniform distribution of pressure around the entry point. Optionally, pressure sensors are distributed around the circumference of the semi-spherical support, which helps the processor monitor the total applied force. This configuration keeps the medical tool stable at the entry point and prevents unwanted punctures.

In an embodiment, the first medical image data from an imaging device is obtained. the first medical image data represents a first volume of the imaged subject and comprises a target and a reference marker. Optionally, the reference marker is registered to an end effector.

FIG. **2** illustrates an embodiment of the surgical robotic system **100** of present disclosure. In this embodiment, the imaging device **103** obtains a first medical image (e.g. 3D medical image) data representing a first volume of the imaged subject **106** and a processor receives the data. The first medical image (e.g., 3D medical image) data representing the first volume of the imaged subject **106** includes a target **109** and a reference marker **107.** Optionally, the first medical image (e.g., 3D medical image) data representing the first volume of the imaged subject includes a target **109,** an entry point (not shown in FIG. **2**), and a reference marker **107.** As shown in FIG. **2****,** reference marker **107** in the first volume is integrated as part of the end effector material or is attached to the end effector. However, the reference marker in the first image volume can be placed on the subject, for example, on the patient.

FIG. **2** shows the end effector **102** is positioned within the imaging gantry alongside the patient, who is on an imaging bed. Reference markers **107** are attached to the end effector, creating a reference marker coordinate system that is registered to the end effector's own coordinate system (Coordinate System **A**). When the imaging device acquires the first medical image, it captures a (e.g., 3D) volume that includes the target as well as the reference marker, representing this information within the imaging device's coordinate system (Coordinate System Rx). Optionally, the medical image data is stored in DICOM (Digital Imaging and Communications in Medicine) format, which includes spatial, and metadata used for coordinating the position and orientation of the image volume relative to the imaging device.

In an embodiment, the end effector's coordinate system **A** is registered with image coordinate system **Rx**. For example, the registration process involves aligning the end effector's coordinate system **A** with the imaging coordinate system **Rx** to ensure accurate spatial correspondence between the medical tool and the target. The positions of the reference markers within the image are identified, allowing their coordinates to be established within the imaging coordinate system. Using, for example, the DICOM metadata, which provides information such as voxel size, slice spacing, and orientation within the image volume, the system can map the reference markers' positions. Since the reference markers have a known spatial relationship to the end effector, this information is used to calculate a transformation matrix, which maps points from the end effector's coordinate system to the imaging coordinate system. By applying this transformation, any position or movement of the medical tool in the end effector's coordinate system **A** can be accurately expressed within the imaging coordinate system **Rx**, allowing for precise medical tool positioning.

In one embodiment, the one or more processors of the surgical robotic system of present disclosure can apply known image registration methods and algorithms such as transformation algorithms (e.g., rigid transformations such as translation and rotation, or non-rigid transformations depending on tissue deformation) to perform the registration. For example, the surgical robotic system uses a rigid transformations algorithm. Optionally, the processor stores this registration information in a memory for further use such as in trajectory calculation. For example, the processor executes the registration algorithm, applying linear algebraic transformations to compute a mapping between the first medical image data and where the robotic arm, the end effector, and/or the medical tool operates.

In another embodiment, target and reference marker, and optionally the entry point in the first medical image are identified by one or more processors of present disclosure.

In one embodiment, upon receiving the first medical image data, the processor decodes and processes the data (e.g. 3D data) to locate and identify one or more features such as the target, entry point, and the reference markers. The processor uses, for example, known image processing algorithms to recognize the features in the image data. Techniques such as edge detection or pattern recognition, or machine learning-based subject detection may be used to automatically identify the reference marker, entry point, and the target. For example, the control unit's processor runs a detection algorithm, which analyzes, for example, the pixel/voxel intensity of the medical image to identify the features.

In another embodiment, the processor receive identity of the target and/or entry point from a display via a display interface or from a memory device. For example, the clinician viewing the first medical image on the display identifies the target and the entry point on the screen of the display. The processors via a display interface receive the identification data of the target and the entry point. In another example, the processors receive the identification data from a memory device.

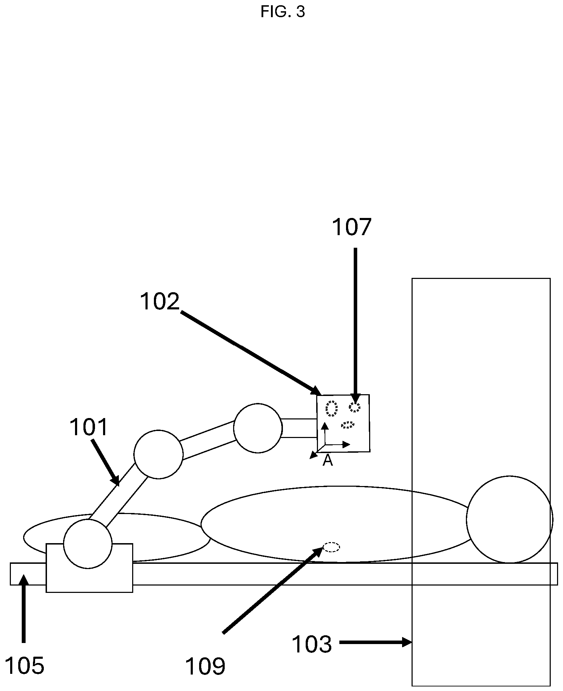

In FIG. **3****,** following the acquisition of the first medical image, the imaging bed **105** carrying the patient, the robotic arm **101,** and the end effector **102,** slides out of the imaging gantry. This movement brings both the end effector **102** and the target **109** outside the gantry, positioning them in a more accessible area for further procedure. Moving the bed outside the gantry provides the medical team with greater physical access to the patient, end effector, and entry point to perform preparatory steps. Additionally, moving the bed outside the gantry may enhance patient safety, as they are not positioned within the enclosed imaging space for extended periods.

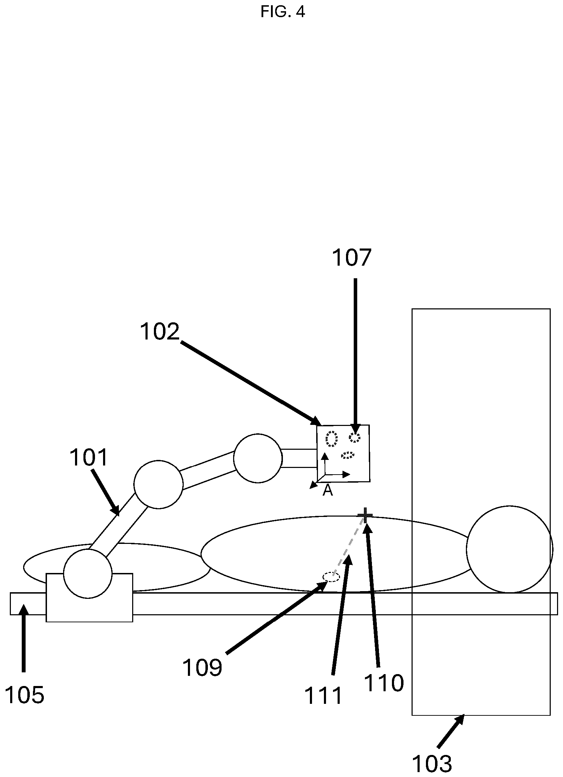

FIG. **4** illustrates a trajectory from an entry point **110** on the subject's surface (e.g., patient's skin) to a target **109** within the subject. While Fig. **4** depicts this trajectory with the patient positioned outside of the imaging gantry, the trajectory can alternatively be determined while the patient is inside the gantry. For example, one or more processes of the system of present disclosure can directly identify the target, (optionally) entry point, and the reference markers in the first medical image and capture the spatial relationship between them and plan the trajectory. Once the trajectory is determined, it can be used outside the gantry, for example, providing access to the entry point.

In an embodiment, one or more processors calculate and determine a trajectory for a medical tool between the identified entry point and the target in the first medical image data based on known methods. Methods like inverse kinematics, spline interpolation, or ray tracing may be used to define the trajectory. For example, the processor calculates the trajectory by solving a series of geometric equations that map the medical tool's entry point to the target. Trajectory is then used by one or more processors to instruct the robotic arm and the end effector on how to position themselves to bring the medical tool such as a needle in precise alignment with the trajectory and thus with the target.

In an embodiment, only the target is identified within the first medical image. The target identification may be performed by a clinician, and the target identification data is received by the processor. Optionally, the target is identified by a processor of the system disclosed herein, as described above. Based on this target location, a trajectory is calculated and determined before identifying the entry point on the subject (e.g., on the patient's skin). For example, the identity of the target within the first medical image is obtained. The first imaging volume captures the target's location in the Rx coordinate frame. Once the target's coordinates are established, the system calculates a potential trajectory extending from the target toward the subject surface (e.g., skin of the patient), considering anatomical structures, angles, any spatial constraints, and safety. The trajectory is determined by analyzing possible paths that ensure the medical tool can reach the target while avoiding critical structures or sensitive areas.

After establishing this trajectory, the system then identifies a suitable entry point on subject (e.g., the patient's skin) where the end effector can position and orient a medical tool for further intervention. This approach provides greater flexibility, as the entry point can be selected to optimize medical tool access and minimize procedural complexity.

In an embodiment, one or more processors optimizes the trajectory (e.g., a path that each medical tool like a needle follows from a point of entry to a target) by considering factors like avoiding undesirable areas, minimizing tissue damage, and maximizing accuracy. The optimization process may involve calculating known algorithms to ensure that the needle follows the most efficient and safest route. In cases where multiple medical tools such as needles need to be actuated (e.g., inserted), the processor also determines the optimal order in which the medical tools should be actuated. Optimization can be based on several factors like minimizing collision between medical tools, ensuring stability of the tissue, or reducing overall procedure time.

In another embodiment, the processor receives the trajectory information from the entry point to the target and/or the information about the order in which the medical tools should be aligned along each trajectory from a display via a user interface or from a memory device.

In one embodiment, the medical tool is positioned and oriented at an entry point along a trajectory reaching the target from the entry point.

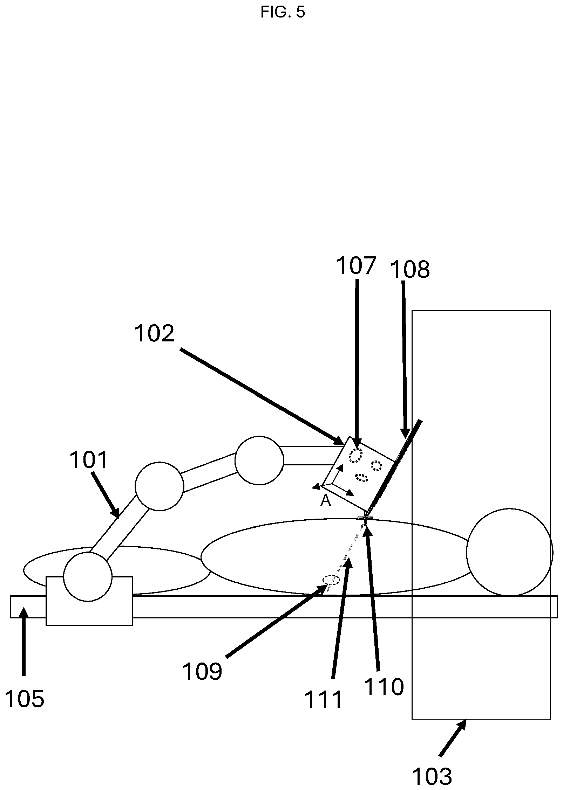

In Fig. **5**, the end effector **102** positions and orients the medical tool **108** (e.g., the needle) at the identified entry point **110** (a point on the patient's skin), aligning it with a trajectory **111** that extends from the entry point **110** to a target **109.**

In one embodiment, as previously described, the end effector applies a controlled amount of force on the subject's surface (e.g. patient's skin), for example via a support arm, ensuring the medical tool is stably positioned at the entry point without substantially penetrating the skin. For example, to prevent unwanted penetration at the entry point, the surgical robotic system of present disclosure includes force-controlled feedback, where a force sensor monitors the pressure applied by the end effector to the needle tip. The force feedback is processed by one or more processors that adjusts the force to ensure it remains below the puncture threshold. Should the applied force approach this threshold, the control system via one or more processors reduces the pressure to maintain safe contact with the entry point surface (e.g., skin). In another example, the surgical robotic system of present disclosure includes an impedance control, in which the end effector's force dynamically adjusts based on the encountered resistance. For example, setting a low impedance, by one or more processors, allows the end effector to apply a pressure when meeting resistance from the entry point (e.g., entry point on the skin), thus enabling the needle to rest stably at the entry point without puncturing. In another example, the surgical robotic system of present disclosure includes ultrasound or optical monitoring systems to detect deformation at the entry point, providing feedback to adjust force as needed, thereby maintaining stable contact without puncturing the surface at the entry point. Another example, pre-defined force thresholds are stored in a memory based on, for example, the skin's resistance properties ensuring the applied force remains within a range that allows stabilization of the needle at the entry point without risking penetration. By employing one or a combination of these exemplary methods, the medical tool is positioned and oriented stably at the entry point in precise alignment with the trajectory, while avoiding or substantially puncture.

In another embodiment, the end effector of present disclosure is configured to position and orient a medical tool such as a needle at an entry point, with controlled force such that the needle achieves an insubstantial penetration. Insubstantial penetration refers to positioning and orienting a medical tool at an entry point on the skin without advancing beyond the skin tissue layers, epidermis, dermis, or hypodermis. Preferably the medical tool does not advance beyond epidermis. The epidermis is the outermost layer of the skin and is avascular and relatively resilient, making it suitable for minor penetration without significant tissue impact. Penetrating with a depth limited to the skin tissue layers such as epidermis or dermis provides a stable anchor point for the needle without deeper tissue engagement.

By applying a controlled force that the medical tool (e.g., needle) is positioned and oriented in the epidermal layers (preferably, epidermis or dermis), the end effector ensures that the medical tool is stabilized at the entry point, achieving sufficient anchoring to align or to maintain alignment along the trajectory, for example, when pivoting at the entry point, while reducing the potential injury to the surrounding tissues.

In one embodiment, an applied force by the end effector is controlled until one or more processors instruct the end effector to actuate (e.g. insert) a medical tool. For example, applied force is controlled to maintain the position and orientation of the medical tool (e.g., needle) at the entry point on the skin along the trajectory until the end effector is instructed by one or more processors to insert the needle along a new trajectory. For example, once the medical tool is positioned and oriented at the entry point, an applied force maintains the medical tool's (e.g. needle's) point of contact with the entry point at the fixed position.

In another embodiment, a second medical image data from an imaging device is received by one or more processors. The second medical image data represent a second volume of the imaged subject comprising the target and the reference marker. Optionally, the reference marker is registered to the end effector. The imaging device used to acquire the first and the second medical image can be the same or different imaging devices, or they can be the same imaging devices but not used in the same procedure. Optionally, the same imaging device within the same procedure (e.g. intervention) is used to acquire the first and the second medical images.

In one embodiment, the second volume of the image data is distinct from the first volume of the image data.

In an embodiment, the second volume of the image data is smaller than the first volume of the imaged data.

In an embodiment, to minimize radiation exposure and procedural time, the second imaged volume is reduced relative to the first imaged volume. Optionally, the first image data can be a higher-resolution, larger volume dataset that encompasses a substantial portion of the region of interest. For instance, the first medical image data can include 160 or more image slices with varying thicknesses, such as 2.5 mm or less. These slices include the region of interest and its surrounding tissues or organs (e.g., lung, kidney, or liver) to allow for accurate planning. The second medical image can, for example, contain 128 or less image slices with varying thicknesses, such as 2.5 mm or less. The second medical image data can have 128, 64, 32, or 16 slices.

In an embodiment, the second medical image (e.g., 3D medical image) data representing a second volume of the imaged subject includes the target and the reference marker, wherein the reference marker is registered to the end effector. Optionally, the second medical image (e.g. 3D image) data representing a second volume of the imaged subject includes the target and the refence marker, wherein the reference marker is registered to the medical tool. Optionally, the second volume further includes the entry point. Optionally, the second medical image (e.g., 3D medical image) data representing a second volume of the imaged subject includes a target, an entry point, and a reference marker, wherein the reference marker is registered to an end effector and/or medical tool.

Optionally, the reference marker in the second medical image data is the same reference marker detectable in the first medical image data. Optionally, only part of the reference marker detectable in the first medical image data is detectable in the second medical image data. However, the reference marker in the second medical image data is used for guiding the end effector to align the medical tool along a trajectory reaching the target from an entry point.

In another embodiment, as shown in Fig. **6****,** the end effector **102** is configured to maintain the medical tool's position at entry point **110** (e.g., patient's skin), aligning with the trajectory **111** to the target **109** for the second medical imaging. This controlled positioning of the end effector allows the medical tool (e.g., needle) to remain stable and aligned with the trajectory while securing the entry point for further steps of the procedure.

In one embodiment, following the acquisition of the second medical image, the location of the target within the second image volume is determined, for example, via one or more processors. Common methods include image segmentation, pattern recognition, or machine learning algorithms trained to distinguish the target from surrounding anatomy. Optionally, one or more processors receive location of the target, for example, from a user interface such as a display or a memory device. Based on the target's determined location, a new trajectory is calculated from the entry point, for example, where the medical tool (e.g., a needle) is positioned on the skin.

In another embodiment, the second medical image is registered with the first medical image. This registration aligns the second image's coordinate system with that of the first, using, for example, the end effector's reference markers, which remains detectable in both images. With the two images registered, the system recalculates the trajectory from the entry point to the updated target position. The surgical robotic system via one or more processors performs registering the second medical image data with the first medical image data. The processor applies known algorithms such as transformation algorithms (e.g., rigid transformations such as translation and rotation, or non-rigid transformations depending on tissue deformation) to register the second medical image data with the first medical image data. For example, a processor computes a rigid registration between the first and second 3D images by utilizing the data contained within the DICOM associated with each image. These DICOM tags include geometric and spatial information such as the position, orientation, and scaling factors of the images.

In an embodiment, a processor computes registration of the second medical image (e.g., 3D medical image) data with the first medical image date via image-to-image registration method. These methods include rigid registration, affine registration, and deformable registration. Exemplary known algorithms for image-to-image registration are mutual information-based registration, normalized cross-correlation, and Demons algorithm (deformable registration).

In an embodiment, a processor computes registration of the second medical image data with the first medical image data using known Demons algorithm. Registration includes, but is not limited to, aligning, or mapping the second image data with the first image data, accounting for differences in orientation, scale, and deformation between the two volumes. The Demons algorithm is a known non-rigid image registration technique that aligns two images by iteratively estimating the local displacement (or deformation) field between them. The algorithm is used, for example, for registering images that have been deformed due to, for example, patient or medical equipment movement during the procedure. Optionally, before applying the Demons algorithm, the second volume is aligned to the first volume. This can be done using a rigid or affine transformation to correct differences in translation, rotation, and scaling between the two volumes. Optionally, the initial displacement field is defined, for example, the initial displacement field (deformation map) is set to zero, indicating that, initially, there is no deformation between the images.

The known Demons algorithm is used, for example, by iteratively estimating a displacement field that maps the second image onto the first image by matching image intensities. According to the present disclosure, the Demons algorithm, optionally, stops after a set number of iterations, for example 1-5 iterations, optionally 2 iterations. The registration information can be saved in a memory for further use according to the present disclosure such as determining the new trajectory.

In an embodiment, according to the present disclosure, both the first and second medical image data contain a reference marker that its coordinate system is registered to coordinate system of the end effector and/or medical tool. For example, the coordinate system or a reference frame of the reference marker is aligned with that of the end effector or the medical tool directly or via an intermediate referential. For example, the reference marker can be directly attached to the end effector or a medical tool or can be part of the material of the end effector or medical tool. In another example, the reference marker is placed on the subject like patient's skin and its coordinate system is registered to coordinate system of the end effector or the medical tool (e.g., the needle), optionally, via an intermediate referential. This allows the processor to maintain an accurate measurement of the spatial relationship between the end effector or medical tool and the target throughout the procedure. For example, during the period between the acquisition of the first medical image and receiving instruction to actuate the medical tool along the trajectory, the end effector, patient, and/or medical tool may undergo movements due to, for example, mechanical drift, vibrations, or adjustments made by the clinician or robotic system. The processor compares the position and orientation data of the end effector and/or medical tool to the reference markers in the second image dataset relative to the first image data. Any detected deviation relative to the target is corrected by one or more processors. Having the reference marker in both the first and second medical image data, any movements are quickly detected and corrected. This means that medical tool actuation such as needle insertion along the trajectory is performed with high accuracy.

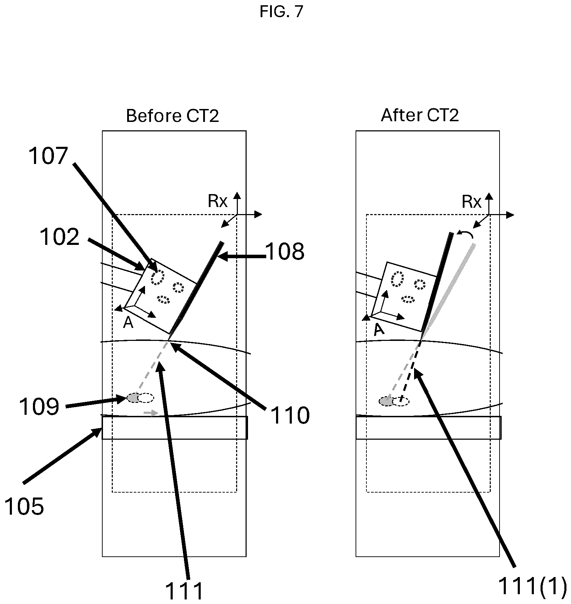

Fig. 7 illustrates the steps following the acquisition of the second medical image (CT2), a new trajectory is **111(1)** determined. In this embodiment, the medical tool **108** (e.g., a needle) already positioned at the entry point and aligned along trajectory **111,** pivots the medical tool at the entry point to align with the newly calculated trajectory reaching the target. This pivoting process ensures that the medical tool remains securely positioned at the entry point while adjusting its angle to precisely align with the new trajectory, considering any changes in the target's position determined in the second medical image. As disclosed above, for example, a gimbal-like joint or spherical bearing integrated within the end effector allows pivoting and multi-directional adjustments. This ensures that the needle can pivot to match the angle required by the new trajectory without disrupting its stable placement on the skin. For example, force sensors within the end effector can control the pivoting force, ensuring the medical tool (e.g. a needle) maintains a stable connection with the entry point while aligning precisely with the new target path.

This rapid pivot-and-insert method ensures that the end effector can dynamically adjust to positional changes of the target, enabling timely medical tool alignment and insertion where the target may shift due to, for example, patient movement, respiration, or factors disclosed herein.

The pivoting of the medical tool at the entry point, as described above, represents just one example of aligning the medical tool with the new trajectory to the target. Alternative methods for adjusting the medical tool's position and/or orientation may also be used to achieve alignment, each with distinct degrees of freedom (DoF) as disclosed above, preferably 3 DoF or less. For instance, the end effector comprises a linear actuator with 2 or 3 DoF to provide reliable medical tool positioning with minimal mechanical complexity, supporting fast and accurate trajectory alignment and insertion.

In an embodiment as illustrated in FIG. **8****,** the surgical robotic system of present disclosure incorporates a tracking system, such as an electromagnetic (EM) tracking system to provide precise alignment and tracking of the end effector during image-guided intervention. In this embodiment, the end effector **102** comprises a rigidly linked EM Sensor **A,** establishing a local coordinate system designated as coordinate system A. Optionally, the EM Sensor A can be linked to the end effector non-rigidly. The term "rigidly linked" means that an object, for example, the sensor is physically attached to another object (such as the end effector or the patient's body) in a way that prevents any relative movement between the objects. This ensures that the spatial relationship between the sensor and the end effector remains constant throughout the procedure. For example, when EM sensor **A** is rigidly linked to the end effector, any movement of the end effector will translate directly to a change in the sensor's position, without any independent motion of the sensor itself.

In an embodiment, sensor **A** configured as an EM emitter or receiver, provides continuous positional data corresponding to the end effector's movement and orientation. Additionally, a reference EM sensor (reference marker), designated Sensor **B,** is rigidly linked to the patient, establishing coordinate system B, which is associated with the patient's position. Optionally, the reference EM sensor B is non-rigidly linked to the patient. Sensor **B** is configured as either an EM emitter or receiver and, optionally, is equipped with radiopaque fiducials, making it visible within the imaging volume. The imaging system itself operates within a fixed coordinate system **Rx**, representing the radiographic reference frame, which defines the spatial orientation of the imaging volume.

As shown in FIG. **8****,** the patient, with Sensor **B** rigidly attached, is positioned within the imaging gantry, and a first medical image is acquired. This first image captures a volume (e.g., 3D volume) containing both the target **109** and Sensor **B.** The processor uses the radiopaque fiducials of Sensor **B** to establish its position within Coordinate System **Rx**. The EM tracking system monitors the positional relationship between Sensor **A** (associated with the end effector's Coordinate System A) and Sensor **B** (associated with the patient's Coordinate System B). By detecting the positions of these sensors within their respective coordinate systems, the EM tracking system computes the relative transformation between Coordinate System A and Coordinate System B. This data is processed by the surgical robotic system's one or more processors, which aligns and maintains alignment between the end effector and the subj ect.

To establish the position of Sensor **A** within the imaging coordinate frame **Rx**, the processor combines, for example, two coordinate transformations. First, it identifies Sensor **B'**s position within Coordinate System **Rx** using, for example, its radiopaque fiducials, captured during the first medical imaging. Second, it utilizes the EM tracking data to determine the relative spatial relationship between Sensor **A** and Sensor **B.** By combining these transformations, the processor accurately locates Sensor **A** and thus the end effector within the imaging coordinate frame **Rx**, aligning the medical tool precisely with the target. This dual-tracking configuration, using both EM tracking and radiopaque fiducials, maintains precise alignment of the medical tool with the trajectory in the intervention. For instance, if movements occur (e.g., patient and/or end effector), the processor recalculates the positions in Coordinate Systems **A, B,** and **Rx**, dynamically updating the alignment to maintain along the trajectory.

Alternative embodiments may employ different localization units, such as optical tracking systems, optionally, in combination with radiopaque fiducials, while maintaining the same principles of coordinate-based alignment and tracking.

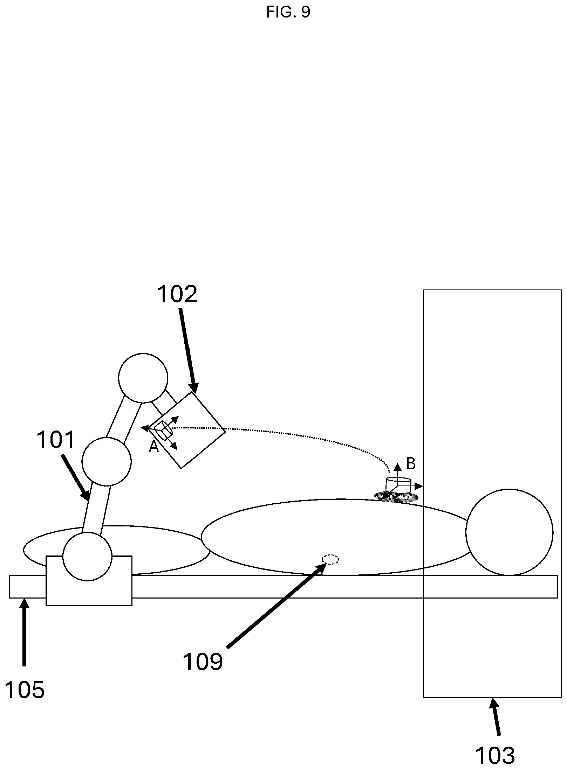

In exemplary FIG. **9****,** following the acquisition of the first medical image, the imaging bed with the sensor **B** rigidly or non-rigidly linked to the patient slides out of the imaging gantry, like the process previously described in relation to FIG. **3****.**

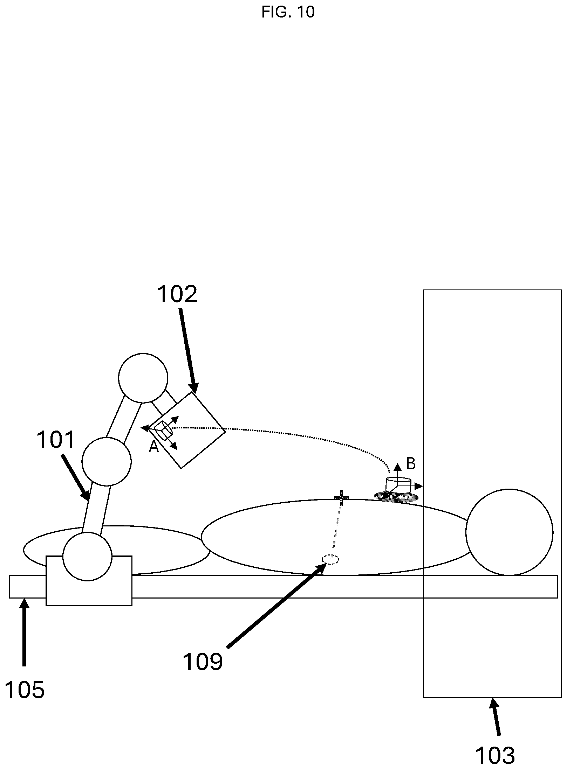

Exemplary FIG. **10** shows a trajectory from the entry point on the subject (e.g., patient's skin) to the target within the patient is determined, for example via one or more processors. Exemplary methods of determining the trajectory and alternatively identifying an entry point via the determined trajectory are described in relation to FIG. **4****.**

For example, as previously disclosed, the first medical image captures a 3D volume containing both the target and the reference EM sensor **B,** which is rigidly or non-rigidly linked to the patient, and it is visible in the imaging data through its radiopaque fiducials. This image is represented within the imaging system's coordinate system, designated as **Rx**. The position of the target is defined within this coordinate system and registered in relation to reference sensor **B.** The EM tracking system continuously tracks both Sensor **A,** which is rigidly or non-rigidly linked to the end effector, and Sensor **B,** attached to the patient. Using the known position of Sensor **B** within the **Rx** coordinate system and the EM data providing the relative positions between Sensor **A** and Sensor **B,** the processor calculates the position of the end effector and entry point relative to the target.

In an alternative embodiment, as described in FIG. **4****,** only the target is identified within the first medical image, and based on this target location, a trajectory is determined before determining the entry point on the subject (e.g., the patient's skin). In this embodiment, the EM tracking system can monitor the movements (e.g. patient and end effector) and ensure the medical tool is aligned and remains aligned with the target once the entry point is identified.

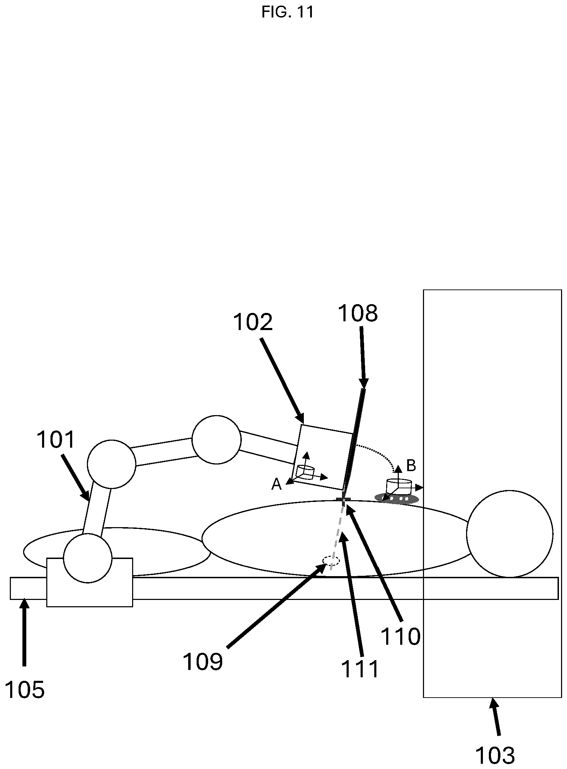

In exemplary Fig. **11****,** the end effector **102** positions and orients a medical tool **108** at an identified entry point **110** on the patient's skin, aligning it with a trajectory **111** that extends from the entry point to a target as described in relation to FIG. **5****.** In this embodiment, the EM tracking system continuously tracks both Sensor **A** and Sensor **B.**

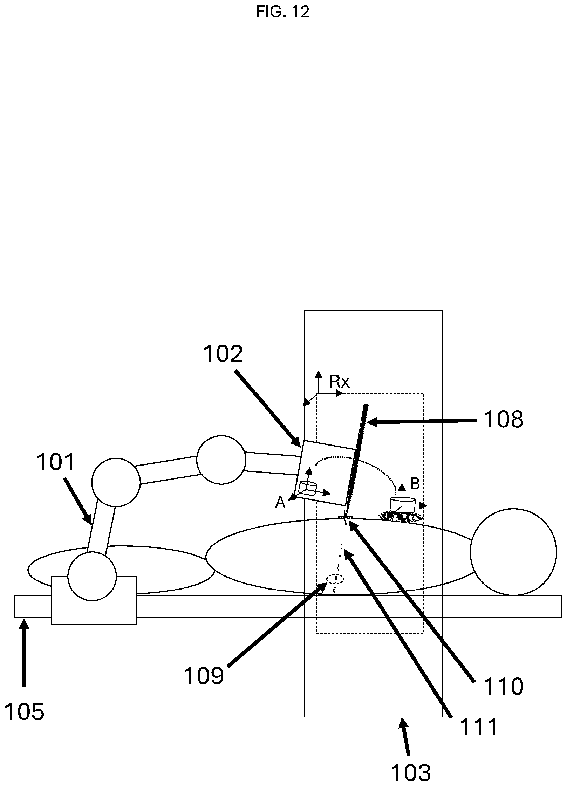

FIG. **12** shows another embodiment as described in FIG. **6** and FIG. 7. As shown, the end effector **102** is configured to maintain medical tool's position at the entry point **110** (e.g., on patient's skin), aligning with the trajectory **111** to the target **109.** The imaging bed moves the patient into the imaging gantry for the second medical imaging. This controlled positioning of the end effector allows the medical tool (e.g., needle) to remain stable and aligned with the trajectory while securing the entry point for further steps of the procedure. The second medical image is acquired. As shown in exemplary FIG. **12****,** the second medical image represents a volume (optionally, smaller than the first medical image volume) and it captures the specific region containing the reference sensor **B** (the reference marker) and the target. In another example, the second medical image contains sensor **B,** entry point, and the target as shown in FIG. **12****.**

In one embodiment, following the acquisition of the second medical image, the location of the target within the second image volume is determined, for example, via the system or one or more processors. Based on the target's determined location, a new trajectory is calculated from the entry point, for example, where the medical tool (e.g., a needle) is positioned to the target. As described in FIG. **7**, after target's location is determined based on the second medical image, one or more processors instruct the end effector to realign and insert the medical tool along the new trajectory from the entry point to the target. For example, as described in FIG. 7, the medical tool pivots at the entry point to align with the new trajectory and then is inserted by instructions received from one or more processors to reach the target.

In an embodiment, as shown in FIG. **12****,** the surgical robotic system utilizes an EM tracking system in combination with radiopaque fiducials to precisely align and track the end effector during the procedure, for example, image-guided intervention. In this example, reference sensor **B** having radiopaque fiducials is visible in the second medical image data. The EM tracking system tracks the relative position between Sensor **A** and Sensor **B,** providing position and orientation data of the end effector relative to the patient. For example, the processors receive two transformations: Sensor **B**'s location in Rx (via radiopaque fiducials) and the relative position of Sensor **A** to Sensor **B** (via EM tracking). By combining these transformations, the processor calculates the exact position of Sensor **A** within the imaging reference frame Rx, ensuring that the end effector remains precisely aligned with the trajectory.

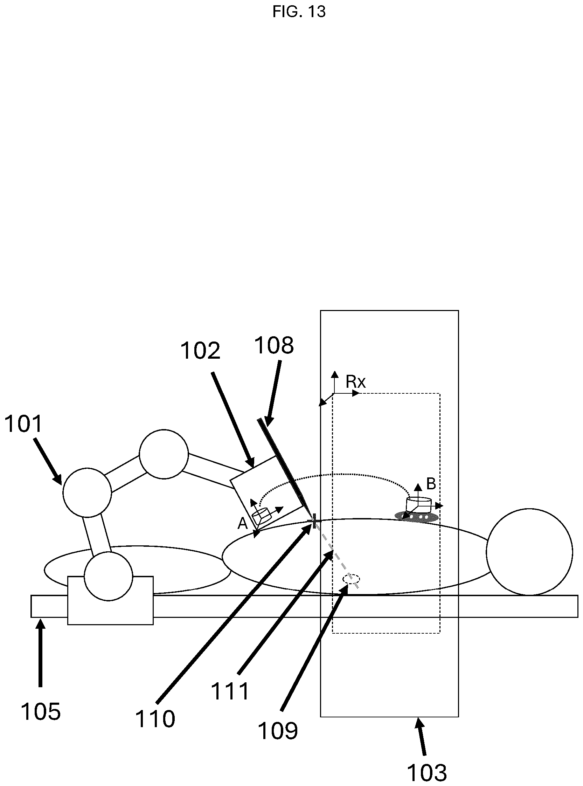

FIG. **13** illustrates another embodiment related to FIG. **12****,** however in this embodiment the entry point **110** remains positioned outside of the gantry and, consequently, outside the field of the second medical image while the target **109** and the reference marker sensor **B** are in the second medical image volume. As shown, the end effector **102** maintains its contact with the entry point outside of the gantry.

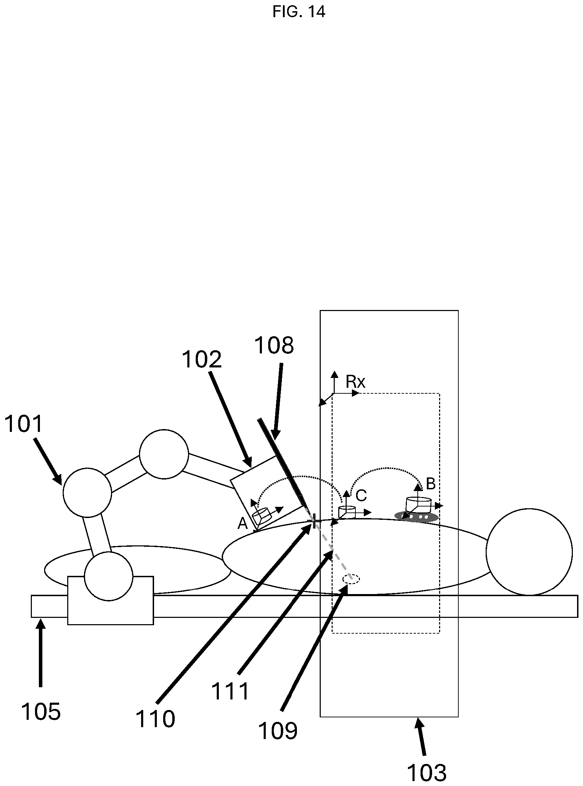

In Fig. **14****,** a patient is positioned within the gantry, and a second medical image, optionally smaller in volume than the first medical image, is acquired by the imaging device. The second medical image comprises the target, reference sensor **B** (reference marker), and an intermediate referential designated as referential sensor **C.** In this example, the end effector, which holds a medical tool at an entry point located outside of the gantry, is equipped with Sensor **A.** Each sensor **A, B,** and **C** has an independent coordinate system, allowing for specific positional references. The imaging system itself operates within the **Rx** coordinate system, which defines the spatial orientation of the imaging volume.

As shown in FIG. **14****,** the second medical image data includes referential sensor **C** and Sensor **B,** establishing a reference within the **Rx** coordinate system. Referential sensor **C** functions as a link that enables the EM tracking system to effectively connect the position and orientation of Sensor **A,** rigidly linked to the end effector, with Sensor **B** rigidly linked to the patient. Optionally, Sensor A and Sensor B are non-rigidly linked to the patient. This bridging mechanism allows the system to determine the precise orientation and position of the end effector relative to the target, despite the end effector and the entry point being outside the gantry.

In an embodiment, the EM tracking system continuously tracks the relative positions of Sensors **A, B,** and intermediate referential **C.** By determining the spatial relationship between Sensor A (end effector) and referential **C,** and then linking referential **C** with Sensor **B** (patient), the system is able to align the end effector's Coordinate System **A** with the imaging coordinate frame **Rx** via the intermediate referential **C.** This intermediate referential provides a positional relationship within the second medical image that compensates for any movement of the patient or end effector during the procedure.

Using referential sensor **C,** the processor calculates the new traj ectory from the entry point to the target. For example, this trajectory is based on the relative positioning data of Coordinate Systems **A, B, C,** and **Rx,** allowing the medical tool to be positioned along the trajectory from the entry point (outside the gantry) to the target. By integrating referential sensor **C** as an intermediary between the Sensor **A** and Sensor **B,** the system maintains the accuracy of trajectory calculations, even when the entry point is outside the gantry and not directly visible in the second medical image data.

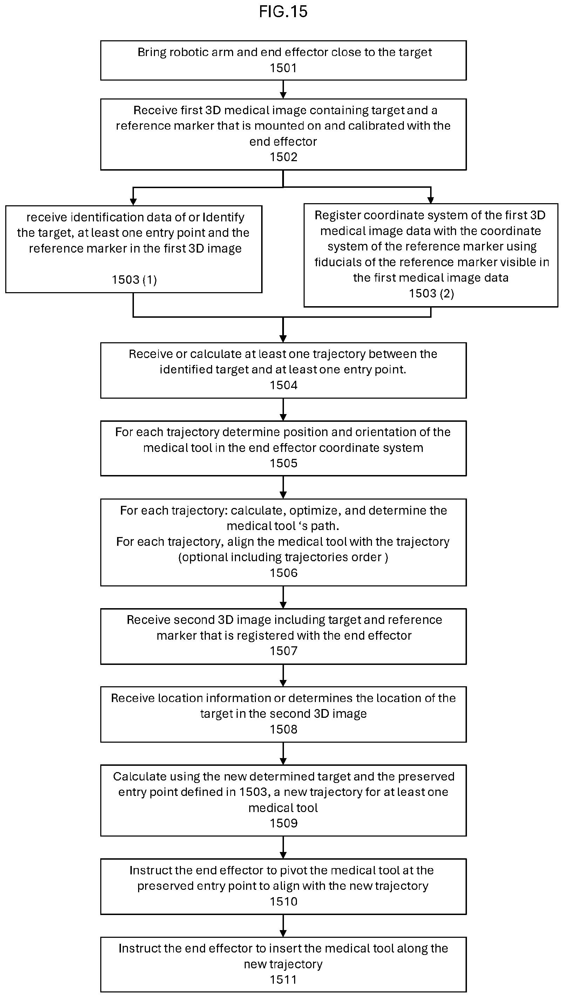

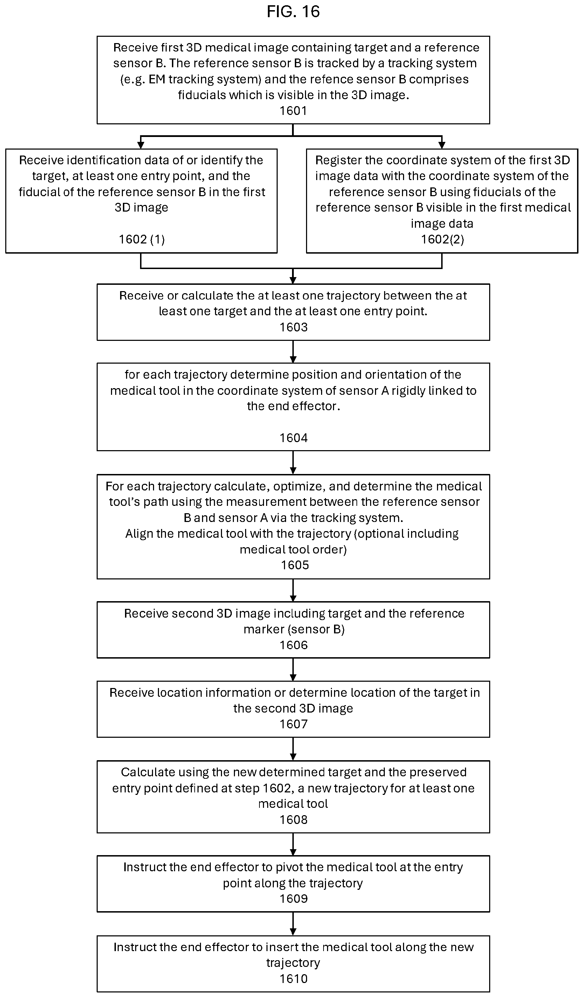

FIG. **15** and FIG. **16** are flowcharts of exemplary methods of present disclosure. In some implementations, one or more process blocks of FIG. **15** and FIG. **16** may be performed by one or more processors of the system. Although FIG. **15** and FIG. **16** show example blocks of the methods, in some implementations, the methods may include additional blocks, fewer blocks, different blocks, differently arranged blocks, additional features, or fewer features in each block than those depicted in FIG. **15** and FIG. **16****.**

FIG. **15** shows a flow chart illustrating an exemplary method according to an embodiment of the present disclosure. The surgical robotic system comprises at least one processor communicatively coupled to the robotic arm and the end effector. At least one processor performs at least one operation of the shown methods. At step **1501,** the robotic arm brings the end effector near the target. For example, the robotic arm brings the end effector within the volume of the first medical image that includes the target. At step **1502,** the processor receives the first medical image data (e.g., 3D image acquired by an imaging device). The first medical image includes a target and a reference marker. Optionally, the reference marker is attached to the end effector and calibrated with the end effector. Steps **1503(1)** and **1503(2)** can be performed simultaneously, before, or after each other or only one of the steps is performed. At step **1503(1),** the processor receives identity of the target, (optionally, entry point as well), and the reference marker in the first medical image data from, for example, a display via a display interface; or the processor identifies the target, (optionally, entry point), and the reference marker in the first medical image data. At step **1503 (2),** the coordinate system of the first 3D medical image data is registered with the coordinate system of the reference marker using fiducials of the reference marker. At step **1504,** the processor receives or determines a trajectory from the identified target and the identified entry point based on the first medical image data. Optionally, the processor determines a trajectory based on the identified target and determines an entry point.

At step **1505,** the processor determines the position and orientation of the medical tool in the end effector coordinate system. At step **1506,** the processor calculates, optimizes, and determines the medical tool's path to be aligned with the trajectory. Optionally, when multiple trajectories are calculated, the order of medical tools to be inserted along each trajectory is calculated and optimized. At step **1507,** a second medical image data is received from an imaging device by the processor (optionally the same imaging device that acquired the first medical image). Optionally, the volume of the second image data is smaller than the volume of the first imaging device. The second image data includes the target, and the reference marker. At step **1508,** the processor receives location information of the target or determines the location of the target in the second medical image from, for example, a display via a display interface. Optionally, the location of the target in the second medical image data is determined by one or more processors. At step **1509,** the processor determines a new trajectory from the target in the second medical image data to the previously identified entry point. At step **1510,** the processor instructs the end effector to align (e.g., via pivoting) the medical tool in contact with surface of the subject at the entry point to align with the new trajectory. At step **1511,** the processor instructs the end effector to actuate (e.g. to insert) the medical tool along the new trajectory.

FIG. **16** shows a flow chart illustrating an exemplary method according to another embodiment of the present disclosure. The disclosed method utilizes a tracking system such as EM tracking system. At least one processor performs one or more operations of the methods.

At step **1601,** a processor receives the first 3D medical image data, which includes a target and a reference sensor **B** (a reference marker). The reference sensor B is rigidly linked to the subject (e.g. a patient's skin). Optionally, reference sensor B is non-rigidly linked to the subject. The reference sensor **B** is an emitter/receiver that is tracked by a tracking system such as EM tracking system. Optionally, reference sensor **B** further comprises an object such as radiopaque material or having recognizable shape that is visible in the medical images.

Steps **1602(1)** and **1602(2)** can be performed simultaneously, before, or after each other or only one of the steps is performed. At step **1602(1),** the processor receives identity of the target, (optionally, entry point), and the reference marker in the first medical image data from, for example, a display via a display interface; or the processor identifies the target, (optionally, entry point), and the reference marker in the first medical image data. At step **1602(2),** the coordinate system of the first 3D medical image data is registered with the coordinate system of the reference sensor B using fiducials of the reference sensor B visible in the first medical image data. At step **1603,** the processor receives or calculates a trajectory from the identified target and the identified entry point based on the first medical image data. Optionally, the processor calculates a trajectory based on the identified target and determines an entry point.

At step **1604,** for each trajectory, the processor determines the position and orientation of the medical tool in coordinate system of Sensor **A** (receiver and/or emitter) that is rigidly or non-rigidly linked to the end effector. In step **1605,** for each trajectory, the processor calculates, optimizes, and determines the medical tool's path using measurements between the reference sensor **B** and a sensor **A** to align with the trajectory. Optionally, when multiple trajectories are calculated, for each trajectory the order of medical tools to be inserted is calculated and optimized by the processor. The measurements include the positional and orientational data gathered by the tracking system to calculate the spatial relationship between reference sensor B and sensor A. For example, measurements include one or more of the following: distance (Linear Measurement), which is the straight-line distance between the reference sensor B and sensor A; orientation (Angular Measurement), which is the relative orientation or angle between the two sensors; positional coordinates, which are coordinates (e.g., X, Y, Z) of each sensor's location in space, which allow for calculating relative positions and directions.

At step **1606,** a second medical image data is received from the imaging device by one or more processors. Optionally, the volume of the second image data is smaller than the volume of the first imaging device. The second image data includes the target, and reference marker sensor **B.** At step **1607,** the location of the target in the second medical image data is determined by one or more processors or the location information of the target is received by one or more processors from, for example, a display or a user interface or from a memory device. At step **1608,** the processor calculates a new trajectory from the target in the second medical image data to the previously identified entry point. At step **1609,** the processor instructs the end effector to pivot the medical tool at the entry point to align with the new trajectory. At step **1610,** the processor instructs the end effector to actuate (e.g. to insert) the medical tool along the new trajectory to reach the target.

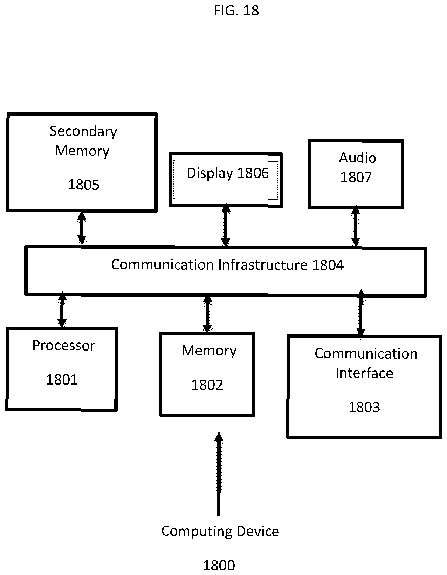

FIG. **18** depicts an exemplary computing device **1800.** Computing device **1800** can be used to implement the processes and to operate the surgical robotic systems according to any of the embodiments of the present disclosure, for example, as shown in FIG. **1-17****.** The following description of the computing device **1800** is provided by way of example only and is not intended to be limiting.

As shown in FIG. **18****,** computing device **1800** includes a processor **1801** for executing software instructions. Although a single processor is shown, the computing device **1800** may also include a multi-processor system. The processor **1801** is connected to a communication infrastructure **1804** for communication with other components of the computing device **1800.** The communication infrastructure **1804** may include, for example, a communications bus or network. The computing device further includes a memory **1802,** such as a random-access memory (RAM), and a secondary memory **1805.** The secondary memory **1805** may include, for example, a storage drive, which may be a hard disk drive, a solid-state drive or a hybrid drive, and/or a removable storage medium such as USB flash drive and a memory card. The memory **1802** and/or removable storage medium in the secondary memory **1805** can include a computer readable storage medium having stored therein computer executable program code instructions and/or data. Optionally, the secondary memory **1805** can allow computer programs or other instructions to be loaded into the computing device **1800.** The computing device **1800** also includes at least one communication interface **1803.** The communication interface **1803** allows software and data to be transferred between computing device **1800** and external devices.

As shown in FIG. **18****,** the computing device **1800** further includes a display interface which performs operations for rendering images and other functions to an associated display **1805.** The computing system can also have an audio interface for performing operations for playing audio content via associated audio speaker(s) **1807.**

For example, computer readable storage media refers to any non-transitory, nonvolatile tangible storage medium that provides recorded instructions and/or data to the computing device **1800** for execution and/or processing. The computer programs are stored in memory **1802** and/or secondary memory **1805.** Computer programs can also be received via the communication interface **1803.** Such computer programs, when executed, enable computing device **1800** to perform one or more features of any of the above disclosed embodiments.

It is to be understood that the embodiment of FIG. **18** is presented merely by way of example and one or more features of the computing device **1800** may be omitted or added.

## Claims

1. A non-transitory computer readable medium storing computer program instructions which, when executed by one or more processors, cause the one or more processors to perform a method comprising:
a) receiving a first medical image data from an imaging device, wherein the first medical image data represents a first volume of an imaged subject, comprising a target within the subject;
b) maintaining alignment of a medical tool along a trajectory extending from an entry point on subject's surface to the target, wherein the medical tool is positioned at the entry point while aligned with the trajectory;
c) receiving a second medical image data from the imaging device while the medical tool maintained its position at the entry point aligned with the trajectory, wherein the second medical image data represents a second volume of the imaged subject, comprising the target and the reference marker;
d) instructing the end effector to insert the medical tool located at the entry point along a new trajectory extending from the entry point on the subject's surface to the target upon receiving an insertion instruction, wherein the new trajectory is determined based on the second medical image.

2. The non-transitory computer readable medium of claim 1, wherein the second volume of the imaged subject is smaller than the first volume of the imaged subject.

3. The non-transitory computer readable medium according to any one of the preceding embodiments, the method comprises: maintaining alignment of the medical tool along the new trajectory by an applied force exerted by the end effector while the medical tool is positioned at the entry point.

4. The non-transitory computer readable medium according to any one of the preceding claims, the method further comprising:
instructing the end effector to pivot the medical tool at the entry point to align with the new trajectory.

5. The non-transitory computer readable medium according to any one of the preceding claims, the method further comprising:
instructing the end effector to maintain an applied force while pivoting the medical tool at the entry point to align with the new trajectory.

6. The non-transitory computer readable medium according to any one of the preceding claims, the method further comprising:
receiving identification data for the target based on the first medical image data;
determining a trajectory extending from the target to an external point on the subject,
wherein the external point is identified as the entry point.

7. The non-transitory computer readable medium according to any one of the preceding claims, the method further comprising:
receiving identification data for the target and the entry point based on the first medical image data;
determining the trajectory between the identified target and the entry point.

8. The non-transitory computer readable medium according to any one of the preceding claims, the method further comprising:
registering coordinate system of the second medical image data with coordinate system of the first medical image data;
determining, on the registered coordinate system of the second medical image data with the coordinate system of the first medical image data, the new trajectory reaching the target.

9. The non-transitory computer readable medium according to any one of the preceding claims, the method further comprising:
registering the coordinate system of the reference marker in the first medical image data and the second medical image data with the coordinate system of the end effector.

10. The non-transitory computer readable medium according to any one of the preceding claims, wherein the reference marker comprises an emitter or receiver trackable by a tracking system and an object that is visible in both the first and second medical images.

11. The non-transitory computer readable medium according to any one of the preceding claims, the method further comprising:
instructing a tracking system to track the reference marker and the end effector with respect to each other, wherein the end effector has a receiver or emitter trackable by the tracking system.

12. The non-transitory computer readable medium according to any one of the preceding claims, wherein the medical tool is a needle.

13. A surgical robotic system, comprising:
an end effector configured to hold and actuate at least one medical tool;
a computing device communicatively coupled to the end effector, wherein the computing device comprises a non-transitory computer readable medium storing computer program instructions which, when executed by one or more processors, cause the one or more processors to perform a method of any one of claim 1-12.

14. The surgical robotic system according to claim 13, wherein the end effector has three or less degrees of freedom.

15. The surgical robotic system according to any one of claim 13-14, wherein the end effector includes a support arm in contact with a surface of a subject and a pivoting mechanism to pivot the medical tool at the entry point.
